# EUROPÄISCHE PATENTSCHRIFT

(11) **EP 0 723 662 B1**
(45) Veröffentlichungstag und Bekanntmachung des Hinweises auf die Patenterteilung: **21.07.1999**
(21) Anmeldenummer: 94926095.4
(22) Anmeldetag: 14.09.1994
(51) Int. Cl.: G01N 33/00, G01N 31/00, G01N 27/12

(54) **SENSOR ZUM NACHWEIS VON STICKOXID**
SENSOR FOR DETECTING NITROGEN OXIDE
DETECTEUR D'OXYDE D'AZOTE

(30) Priorität: 12.10.1993 DE 4334672
(43) Veröffentlichungstag der Anmeldung: 31.07.1996
(73) Patentinhaber: ROBERT BOSCH GMBH, 70442 Stuttgart (DE)
(72) Erfinder: POTTHAST, Heidrun, D-70825 Korntal-Muenchingen (DE); SCHUMANN, Bernd, D-71277 Rutesheim (DE)
(74) Vertreter: Röser, Peter, Dipl.-Ing.
(86) Internationale Anmeldenummer: DE9401051
(87) Internationale Veröffentlichungsnummer: WO9510774

(56) Entgegenhaltungen:
- EP-A- 0 313 390
- EP-A- 0 517 366
- DE-A- 2 648 373
- DE-A- 3 624 217
- US-A- 4 840 913
- US-A- 4 927 517
- JOURNAL OF MATERIALS SCIENCE, Bd.25, Nr.5, Mai 1990, LONDON GB Seiten 2632 - 2636 Y.SADAOKA ET AL 'Effect of NO2 in air on the electrical conductance of In2O3 films with and without added ZnO prepared by R.F. Sputtering.'

## Beschreibung

### Stand der Technik

Die Erfindung betrifft einen Sensor zum Nachweis von Stickoxiden (NO, NO₂, N₂O₄) nach der Gattung des Anspruchs 1. Es ist bekannt, zur Bestimmung von spezifischen Komponenten eines Prüfgases Sensoren zu verwenden, deren sensitives Element ein Halbleitermaterial ist, dessen elektrischer Widerstand sich bei Berührung mit der spezifischen Gaskomponente ändert. Verwendung finden Sensoren dieser Art insbesondere zur Bestimmung des Sauerstoffgehalts in Abgasen, zum Beispiel aus Verbrennungskraftmaschinen, aber auch zur Bestimmung von Methan, Kohlenmonoxid oder Alkohol. Als Halbleitermaterialien werden insbesondere halbleitende Metalloxide, wie Zinnoxid (SnO₂), Zinkoxid (ZnO), Titanoxid (TiO₂), oder Wolframoxid (WO₃) je nach Zweck eingesetzt.

Hergestellt werden diese bekannten Gassensoren üblicherweise in Dick- oder Dünnschichttechnik. Auf ein isolierendes, vorzugsweise keramisches Substrat, zum Beispiel aus Aluminiumoxid (Al₂O₃) werden Leiterbahnen, mittels derer später die Widerstandsänderung bestimmt wird, sowie das halbleitende Oxid aufgebracht. Um einerseits die Empfindlichkeit des Sensors - diese ist temperturabhängig - zu erhöhen, und andererseits die Aufrechterhaltung des dynamischen Gleichgewichts von Adsorption und Desorption zu gewährleisten, ist es üblich, das Substrat mit der Sensoranordnung zu beheizen. Hierzu erforderliche Heizeinrichtungen können nach bekannten Vorschlägen zum Beispiel auf der Unterseite des Substrates angeordnet sein - wenn an der Oberseite die Sensoranordnung aufgebracht ist -, oder sie können in das Substrat integriert oder zwischen Substrat-Oberfläche und Sensoranordnung angeordnet sein.

Bekannt ist ein solcher Sensor zum Beispiel aus der EP-OS 313 390. Bei dem darin beschriebenen Sensor befinden sich Heizungseinrichtung und Sensoranordnung auf einer Seite eines Substrates aus Aluminiumoxid (Al₂O₃). Als halbleitendes Material wird Zinnoxid (SnO₂) für Methangas, Woframoxid (WO₃) für Kohlenmonoxid oder Lanthan-Nickeloxid (LaNiO₃) zur Detektion von Alkohol vorgeschlagen.

Ein Sensor, bei welchem die Heizeinrichtung in das Substrat integriert ist, ist aus der DE-OS 36 24 217 bekannt. Als Material für das Substrat wird wiederum Aluminiumoxid (Al₂O₃) vorgeschlagen, die gassensitve Halbleiterschicht besteht in diesem Fall aus einem porösen Titandioxid (TiO₂), welches mit einem weiteren Metalloxid angereichert ist. Der beschriebene Sensor ist insbesondere zur Regelung des Luft/Brennstoffverhältnis in einem Abgas durch Messung des Sauerstoffgehaltes vorgesehen.

Zwar haben sich diese bekannten Sensoren auf Halbleiteroxidbasis zur Erfassung von CO, H₂ und Kohlenwasserstoffen praktisch bewährt, zum Nachweis von Stickoxid (NO) bzw. Stickstoffdioxid (NO₂) sind sie jedoch ungeeignet. Gerade deren Detektion ist aber von erheblicher Bedeutung zum Nachweis von Dieselabgasen. In solchen befindet sich vor allem Stickoxid (NO), welches sich nach Minuten nahezu vollständig in Stickstoffdioxid (NO₂) bzw. Distickstofftetroxid (N₂O₄) umwandelt. Wegen der unterschiedlichen Einwirkung von Stickstoffmonoxid (NO) bzw. Stickstoffdioxid (NO₂) auf das halbleitende Oxid reagiert dieses mit einem komplizierten Widerstandsverhalten, welches nicht zweckgemäß ausgewertet werden kann. Zum Nachweis von Dieselabgasen ist es deshalb bekannt, Phthalocyaninschichten einzusetzen, welche im Abgas enthaltene NOₓ-Moleküle nachweisen. Der Nachweis von Dieselabgasen wiederum kann zum Beispiel dazu dienen, bei Lüftungen oder Klimaanlagen die Zufuhr von Außenluft, in welcher Dieselabgase festgestellt wurden, in Räume oder Fahrzeuge durch eine Steuerklappe zu unterbrechen. Von besonderem Interesse ist die genaue Kenntnis des Anteils der Stickoxide (NO, NO₂ ...) in den Abgasen zur Überwachung und Regelung von Brennkraftmaschinen oder Feuerungsanlagen. Dies gilt vor allem dann, wenn Katalysatoren und andere Verfahren zur Optimierung der Verbrennung und Reduzierung der unerwünschten Abgasanteile wie NO, CO oder Kohlenwasserstoffe eingesetzt werden.

Aufgabe der vorliegenden Erfindung ist es, einen Sensor sowie ein Verfahren zu seiner Herstellung anzugeben, welcher einfach aufgebaut ist und eine hinreichend gute Bestimmung des Anteils an Stickoxiden in einem Prüfgas gestattet.

Diese Aufgabe wird gelöst durch einen Sensor bzw. ein Verfahren mit den Merkmalen des Anspruchs 1. Der erfindungsgemäße Sensor ist mit grundsätzlich bekannten Techniken und dadurch kostengünstig herstellbar. Er zeigt beim Einsatz schnell und mit guter Empfindlichkeit stickoxidhaltige Gase an, ohne von gleichzeitig vorhandenen CO, H₂ oder CHₓ-Bestandteilen beeinflußt zu werden. Er eignet sich deshalb besonders zum Einsatz in Kraftfahrzeugen zur Steuerung der Innenraumbelüftung, welche er gegebenenfalls unterbricht, wenn Dieselabgase in den Innenraum eindringen.

Vorteilhafte Weiterbildungen und zweckmäßige Ausgestaltungen des erfindungesgemäßen Sensors bzw. des Verfahrens zu seiner Herstellung ergeben sich aus den Unteransprüchen.

Durch Beschichtung des Sensors mit Palladium (Pd) kann die Stickstoffdioxid (NO₂)-Empfindlichkeit im Hinblick auf die vorgegebene Verwendung eingestellt werden. Eine zweite Beschichtung des Sensors mit Platin (Pt) und/oder Rhodium (Rh) wird zweckmäßig dazu verwendet, die Desorption des gebildeten Stickstoffdioxids (NO₂) zu unterstützen sowie die Anstiegsgeschwindigkeit und Differenzierung des Signals bei unterschiedlichen Stickstoffdioxid (NO₂)-Konzentrationen zu verbessern. Der Sensor kann dadurch niedrige, im Straßenverkehr übliche NO/NO₂-Konzentrationen bei einem stabilen Grundwiderstand detektieren, ohne daß es zu einer ständigen Erhöhung des Widerstands und einer Drift kommt.

Von Vorteil ist weiterhin, daß der vorgeschlagene Sensor im Gegensatz zu beispielsweise organischen Phtalocyaninsensoren bis zu Temperaturen von 700° C ausgeheizt werden kann, um ihn periodisch zu reinigen und die Oberfläche zu stabilisieren.

Der vorgeschlagene Sensor ist auch gut geeignet zur summarischen Bestimmung des NO/NO₂-Gehaltes im Abgas von Feuerungsanlagen in Abhängigkeit von dessen Sauerstoffgehalt.

Nachfolgend wird der vorgeschlagene Sensor anhand von in der Zeichnung dargestellten Ausführungsbeispielen näher erläutert.

Es zeigen Figuren 1 bis 3 Draufsichten auf einen erfindungsgemäßen Sensor, Figur 4 eine Sicht desselben Sensors von der Unterseite, Figur 5 eine weitere Ausführung des erfindungsgemäßen Sensors in Seitenansicht.

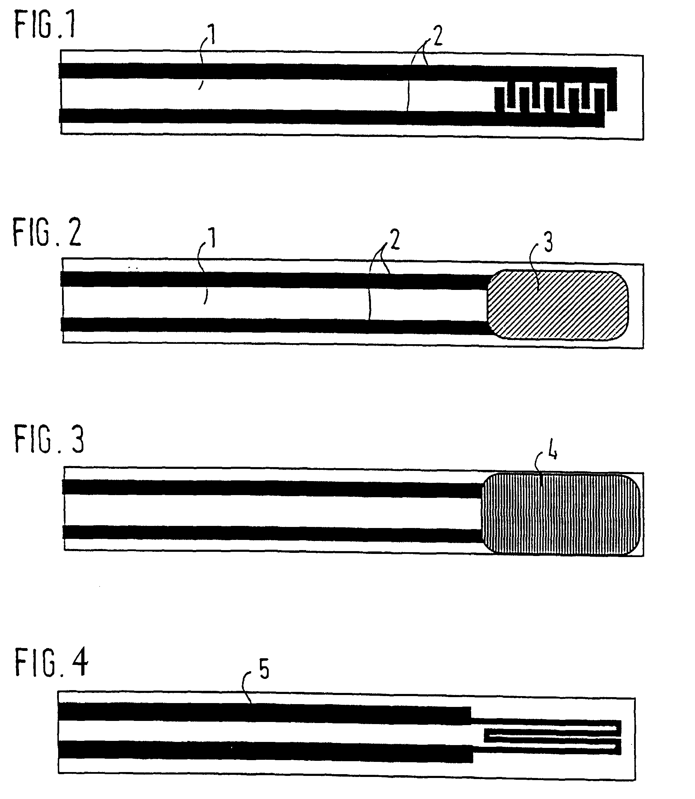

Figur 1 zeigt eine Draufsicht auf einen vorgeschlagenen Sensor, der stäbchenförmig ausgeführt wurde. Er besteht aus einem Substrat aus einem elektrisch isolierenden und wärmebeständigen Material, vorzugsweise Aluminiumoxid (Al₂O₃), auf welches in Dickschichttechnik die weiteren den Sensor bildenden Bestandteile aufgebracht sind. Auf einer hier als Oberseite bezeichneten Oberfläche des Substrates sind zwei Leiterbahnen 2 aufgebracht, welche an ihren Kopfenden kammartig ineinander verzahnt sind.

Im Bereich der verzahnten Enden ist über die Leiterbahnen 2 zweckmäßig in einer Dicke 10 bis 500 µm eine halbleitende Metalloxidschicht 3 aufgebracht. Sie besteht aus Indiumoxid (In₂O₃) oder Zinnoxid (SnO₂), welchem in Konzentrationen von 0,005 bis 0,05 Mol/% ein die Leitfähigkeit erhöhendes Dotierungselement beigemischt ist. Als Dotierungselement kann Tantal (Ta), Niob (Nb), Antimon (Sb) oder Wolfram (W), bei Indiumoxid (In₂O₃) auch Zinn (Sn), Titan (Ti) oder Cer (Ce) verwendet werden. Zur Einschränkung des Kristallitwachstums, damit zur Erhöhung der Empfindlichkeit und zur Verbesserung der Alterungsbeständigkeit der Sensoren kann zusätzlich noch ein 2-wertiges Element, insbesondere Magnesium (Mg), Barium (Ba), Calcium (Ca), Strontium (Sr), Zink (Zn) oder ein 3-wertiges Element wie etwa Aluminium in Aluminiumoxid (Al₂O₃) in Konzentrationen von 0,001 bis 45% in dem Metalloxid enthalten sein. Das Metalloxid der Schicht 3 ist weiterhin mit einem Edelmetallzusatz imprägniert. Dieser besteht insbesondere aus Palladium (Pd) mit Zusätzen von Platin (Pt) und/oder Rhodium (Rh) in einer Konzentration von 0.001 bis 0.5 Mol%. Dabei dient das Palladium (Pd) zur Einstellung der Stickstoffdioxid (NO₂)-Empfindlichkeit. Der Platin bzw. Rhodium-zusatz erleichtert die Desorption des gebildeten Stickstoffdioxides (NO₂). Ferner verbessert er die Anstiegsgeschwindigkeit sowie die Differenzierung des Signals bei unterschiedlichen Stickstoffdioxid (NO₂)-Konzentrationen. Dadurch kann der Sensor niedrige, verkehrsübliche Stickoxid/Stickstoffdioxid-Konzentrationen bei einem stabilen Grundwiderstand detektieren, ohne daß es zu einer ständigen Erhöhung des Widerstandes und einer Drift kommt.

Über die Metalloxidschicht 3 ist in einer Stärke von zweckmäßig 10 bis 100 µm eine Konverterteilschicht 4 aufgebracht. Sie besteht vorzugsweise aus Aluminiumoxid (Al₂O₃), Titanoxid (TiO₂), Zirkoniumoxid (ZrO₂) oder Siliziumoxid (SiO₂) mit einem Platingehalt von 0,01 bis 20%. Ihre Herstellung basiert auf pulverförmigem Ausgangsmaterial, das mit dem Platin bzw. dem verwendeten Edelmetall imprägniert oder dem platinhaltiges Metallpulver beigemischt wird. Auf der gegenüberliegenden Seite des Substrates 1, welche hier, entsprechend der Darstellung der Figuren 1 bis 3 als Unterseite bezeichnet wird, befindet sich eine Heizungsanordnung 5, welche in Figur 4 als Draufsicht dargestellt ist. Sie besteht im wesentlichen aus einer im Bereich unterhalb der Metalloxidschicht 3 mäandernden Leiterbahn.

Funktionsprinzip des erfindungsgemäßen Sensors ist, das im Prüfgas enthaltende Stickoxid zunächst in Stickstoffdioxid (NO₂) umzuwandeln und dieses dann der sensitiven Metalloxidschicht 3 zuzuführen. Die Umwandlung erfolgt in der Konverterschicht 4 durch Oxidation des Stickoxids (NO) mit dem Luftsauerstoff zu Stickstoffdioxid (NO₂) oder Distickstofftetroxid (N₂O₄). Die Konverterschicht 4 erfüllt dabei gleichzeitig die Funktion, das noch nicht konvertierte Stickstoffmonoxid (NO) von der hableitenden Metalloxidschicht 3 fernzuhalten. Ihre Dicke ist entsprechend so zu wählen, daß kein Stickstoffmonoxid (NO) an die Metalloxidschicht gelangt. Ferner dient die Konverterschicht 4 dazu, im Prüfgas enthaltende brennbare Bestandteile wie Kohlenmonoxid (CO), Wasserstoff (H₂) oder Kohlenwasserstoffe mit dem im Prüfgas enthaltenen Sauerstoff zu oxidieren. Dadurch beeinflussen brennbare und/oder oxidierbare Bestandteile des Prüfgases die elektrischen Eigenschaften des halbleitenden Metalloxids, und damit das Meßergebnis nicht mehr. Eine weitere Funktion erfüllt die Konverterschicht 4, indem sie das Stickoxid (NO) bzw. Stickstoffdioxid (NO₂) bis zu einer durch die Verweilzeit der Gasmoleküle über der halbleitenden Metalloxidschicht 3 bestimmten Menge speichert. Dadurch wird bei kleinen Stickoxid (NO)-Konzentrationen die Empfindlichkeit des Sensors erhöht, indem mehr NO in NO₂ konvertiert wird.

Das an die - n-leitende - Metalloxidschicht 3 gelangende Stickstoffdioxid (NO₂) bindet bei Kontakt mit der Oberfläche aufgrund seiner Dipoleigenschaften Elektronen und bewirkt so eine Zunahme des elektrischen Widerstandes der Metalloxidschicht 3, die in bekannter Weise gemessen wird. Hierzu wird zum Beispiel über die Leiterbahnen 2 ein Konstantstrom angelegt; die nach Änderung des Widerstandes der Metalloxidschicht 3 erforderlichen Änderungen der zur Aufrechterhaltung des Konstantstroms benötigten Spannungen werden gemessen.

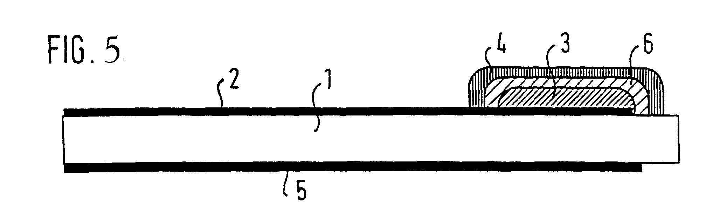

Um die Empfindlichkeit des Sensors zu erhöhen, wird er von der Unterseite des Substrates 1 her mittels der Heizanordnung 5 auf eine Temperatur von etwa 180° bis 400° C geheizt. Durch die Erwärmung erhöht sich die Leitfähigkeit in der Metalloxidschicht 3. Ferner unterstützt das Heizen die Aufrechterhaltung des dynamischen Gleichgewichtes von Adsorption und Desorption. Zudem beschleunigt es die Oxidation der im Prüfgas enthaltenen brennbaren Bestandteile. Figur 5 zeigt beispielhaft den erfindungsgemäßen Sensoraufbau mit zwei Konverterteilschichten. Zwischen der Metalloxidschicht 3 und einer ersten Konverterteilschicht 4 ist eine zweite Konverterteilschicht 6 angebracht. Sie besteht wie die erste Konverterteilschicht 4 zum Beispiel aus Alumuniumoxid (Al₂O₃), Titanoxid (TiO₂), Zirkoniumoxid (ZrO₂) oder Siliziumoxid (SiO₂), weist aber vorzugsweise einen verringerten Edelmetallgehalt, etwa an Platin, von 0,001 bis 2% auf. Ihre Dicke beträgt zweckmäßig 10 bis 500 µm. Um Haftung und thermische Ausdehnung der Konverterteilschicht 6 an die der Metalloxidschicht 3 anzupassen, können auch Mischungen der genannten Oxide mit Zinnoxid (SnO₂) oder Indiumoxid (In₂O₃) eingesetzt werden.

Durch die Konverterteilschicht 6 wird sichergestellt, daß alle Stickoxide (NO) in Stickstoffdioxide (NO₂) bzw. Distickstofftetroxid (N₂O₄) konvertiert werden und keine Stickoxide (NO) zu der Metalloxidschicht 3 gelangen. Die Verweilzeit der Stickoxid-(NO) bzw. Stickstoffdioxid-(NO₂)-Moleküle über der Metalloxidschicht 3 wird erhöht. Das Einbringen der Konverterteilschicht 6 führt zu einer Funktionstrennung derart, daß in der ersten Konverterschicht 4 im wesentlichen die Oxidation der brennbaren Bestandteile des Prüfgases erfolgt, während die Konvertierung des Stickstoffmonoxids (NO) in der Konverterteilschicht 6 erfolgt. Ein Aufbau mit zwei Konverterschichten 4 bzw. 6 ist insbesondere bei hohen Konzentrationen von brennbaren Bestandteilen im Prüfgas von großem Vorteil.

Sowohl die Konverterteilschicht 6 wie auch die Konverterteilschicht 4 können ferner Bestandteile zur Beschleunigung der Oxidationsprozesse enthalten, wie zum Beispiel Ceroxid (CeO₂).

Ein weiteres Ausführungsbeispiel geht von der Ausführungsform gemäß Figur 5 aus. Dabei wird über die Sensorschichtanordnung 1 bis 4 eine poröse Schicht, z. B. aus Aluminiumoxid (Al₂O₃) und/oder Glaspulver mit einer Dicke von 100 bis 500 µm aufgebracht. Auf der Oberseite der porösen Schicht wird dann die Heizungsanordnung 5 plaziert, welche die Sensoranordnung 1 bis 4 somit von der Oberseite her erwärmt. Zweckmäßig wird wenigstens einer der Konverterschichten 4 bzw. 6 ein Porenbildner beigemischt, so daß die Temperatur der Metalloxidschicht 3 niedriger gehalten werden kann, als die Temperatur der Konverterschichten 4 bzw. 6. Eine derartige Anordnung erlaubt den Betrieb des Sensors bei Temperaturen bis zu 450° C.

## Patentansprüche

1. Sensor zum Nachweis von Stickoxiden (NO, NO₂, N₂O₄) in einem Prüfgas mit einer auf einem vorzugsweise keramischen Substrat (10) aufgebrachten halbleitenden Metalloxidschicht (3), deren elektrischer Widerstand eine Aussage über die Konzentration von Stickoxiden (NO, NO₂, N₂O₄) in dem Prüfgas liefert, mit
- einer auf die Metalloxidschicht (3) aufgebrachten Konverterschicht aus einem die Oxidation brennbarer Bestandteile des Prüfgases bewirkenden Material, die im Prüfgas enthaltenes Stickstoffmonoxid (NO) in Stickstoffdioxid (NO₂) oder Distickstofftetroxid (N₂O₄) konvertiert, welches anschließend zu der Metalloxidschicht (3) gelangt,
- sowie einer Heizanordnung (5), die die Metalloxidschicht (3) und Konverterschicht erwärmt,
dadurch gekennzeichnet, daß
die Konverterschicht zwei jeweils auf einem keramischen Grundmaterial aufbauende Teilschichten (4,6) aufweist, denen in voneinander verschiedener Konzentration jeweils ein katalytisch wirksamer Anteil von Edelmetall(en) der Platingruppe zugesetzt ist.

2. Sensor nach Anspruch 1, dadurch gekennzeichnet, daß das keramische Grundmaterial der Konverterschicht(-en) (4,6) Titanoxid (TiO₂) und/oder Zirkoniumoxid (ZrO₂) und/oder Siliziumoxid (SiO₂) und/oder Aluminiumoxid (Al₂O₃) ist.

3. Sensor nach Anspruch 1, dadurch gekennzeichnet, daß die Metalloxidschicht (3) aus einem halbleitenden Metalloxid, insbesondere Indiumoxid (In₂O₃) oder Zinnoxid (SnO₂), besteht, welchem in einer Konzentration von 5 x 10⁻⁴ bis 5 x 10⁻² Mol% ein die Leitfähigkeit erhöhendes Dotierungselement beigemischt ist.

4. Sensor nach Anspruch 3, dadurch gekennzeichnet, daß das Dotierungselement Tantal (Ta), Niob (Nb), Antimon (Sb), Wolfram (W), beim Indiumoxid (In₂O₃) Zinn (Sn), Titan (Ti) oder Cer (Ce) ist.

5. Sensor nach Anspruch 1, dadurch gekennzeichnet, daß die Metalloxidschicht (3) zusätzlich mindestens ein zweiwertiges Element der Hauptgruppe II und/oder ein dreiwertiges Element der Hauptgruppe III und/oder ein dreiwertiges Element der seltenen Erden als Oxid in einer Konzentration von 0.001 bis 45 Gewichtsprozent erhält.

6. Sensor nach Anspruch 5, dadurch gekennzeichnet, daß das zusätzliche Element Magnesium (Mg), Barium (Ba), Calcium (Ca), Strontium (Sr), Zink (Zn) oder Aluminium in Aluminiumoxid (Al₂O₃) ist.

7. Sensor nach Anspruch 1, dadurch gekennzeichnet, daß die Metalloxidschicht (3) Palladium (Pd) mit einem Zusatz von Platin (Pt) und/oder Rhodium (Rh) und/oder eine Legierung daraus in einer Konzentration von 0.001 bis 5 Mol% enthält.

8. Sensor nach Anspruch 1, dadurch gekennzeichnet, daß die Konverterteilschicht (4) in einer Konzentration von 0.01 bis 20 Gewichtsprozent, Platin (Pt) und/oder Rhodium (Rh), und/oder Palladium (Pd) oder eine Legierung daraus enthält.

9. Sensor nach Anspruch 1, dadurch gekennzeichnet, daß auf die Konverterteilschicht (4) eine poröse Schicht aufgebracht ist auf deren Oberseite die Heizanordnung (5) angeordnet ist.

10. Sensor nach Anspruch 1, dadurch gekennzeichnet, daß die eine Konverterteilschicht (6) einen gegenüber der anderen Konverterteilschicht (4) geringeren Edelmetallanteil aufweist.

## Claims

1. Sensor for detecting nitrogen oxides (NO, NO₂, N₂O₄) in a gas to be examined, having a semiconducting metal oxide layer (3) which is applied to a preferably ceramic substrate (10) and whose electrical resistance provides information about the concentration of nitrogen oxides (NO, NO₂, N₂O₄) in the gas to be examined, having
- a converter layer which is made of a material which causes the oxidation of combustible components of the gas to be examined, is applied to the metal oxide layer (3) and converts the nitrogen monoxide (NO) contained in the gas to be examined into nitrogen dioxide (NO₂) or dinitrogen tetroxide (N₂O₄), which is subsequently transported to the metal oxide layer (3),
- and a heating arrangement (5) which heats the metal oxide layer (3) and converter layer,
characterized in that the converter layer has two sublayers (4, 6) which are respectively based on a ceramic base material and to which a catalytically active fraction made up of one or more noble metals from the platinum group is respectively added in a different concentration.

2. Sensor according to Claim 1, characterized in that the ceramic base material of the converter layer (s) (4, 6) is titanium oxide (TiO₂) and/or zirconium oxide (ZrO₂) and/or silicon oxide (SiO₂) and/or aluminium oxide (Al₂O₃).

3. Sensor according to Claim 1, characterized in that the metal oxide layer (3) consists of a semiconducting metal oxide, in particular indium oxide (In₂O₃) or tin oxide (SnO₂), to which a conductivity-increasing dopant is added in the concentration of 5 x 10⁻⁴ to 5 x 10⁻² mol%.

4. Sensor according to Claim 3, characterized in that the dopant is tantalum (Ta), niobium (Nb), antimony (Sb), tungsten (W), in the case of indium oxide (In₂O₃), tin (Sn), titanium, (Ti) or cerium (Ce).

5. Sensor according to Claim 1, characterized in that the metal oxide layer (3) additionally contains at least one divalent element from main group II and/or a trivalent element from main group III and/or a trivalent rare-earth element as oxide in a concentration of 0.001 to 45 per cent by weight.

6. Sensor according to Claim 5, characterized in that the additional element is magnesium (Mg), barium (Ba), calcium (Ca), strontium (Sr), zinc (Zn) or aluminium in aluminium oxide (Al₂O₃).

7. Sensor according to Claim 1, characterized in that the metal oxide layer (3) contains palladium (Pd) with added platinum (Pt) and/or rhodium (Rh) and/or an alloy thereof in a concentration of 0.001 to 5 mol%.

8. Sensor according to Claim 1, characterized in that the converter sublayer (4) contains, in a concentration of 0.01 to 20 per cent by weight, platinum (Pt) and/or rhodium (Rh), and/or palladium (Pd) or an alloy thereof.

9. Sensor according to Claim 1, characterized in that a porous layer , on the upper side of which the heating arrangement (5) is arranged, is applied to the converter sublayer (4).

10. Sensor according to Claim 1, characterized in that one converter sublayer (6) has a lower noble metal fraction than the other converter sublayer (4).

## Revendications

1. Détecteur pour déceler des oxydes d'azote (NO, NO₂, N₂O₄) dans un gaz de contrôle avec une couche d'oxyde métallique (3) semi-conducteur appliquée de préférence sur un substrat en céramique (10), couche dont la résistance électrique donne une information relative à la concentration en oxydes d'azote (NO, NO₂, N₂O₄) dans le gaz de contrôle, comprenant :
- une couche de conversion appliquée sur la couche d'oxyde métallique (3), cette couche de conversion étant en une matière réalisant l'oxydation de composants combustibles contenus dans le gaz de contrôle, la conversion du monoxyde d'azote (NO) du gaz de contrôle en dioxyde d'azote (NO₂) ou tétraoxyde d'azote (N₂O₄) qui arrive finalement sur la couche d'oxyde métallique (3),
- ainsi qu'un dispositif de chauffage (5) qui chauffe la couche d'oxyde métallique (3) et la couche de conversion,
caractérisé en ce que
la couche de conversion comporte deux couches partielles (4, 6) que l'on réalise chaque fois sur un support de base céramique et auxquelles on ajoute dans des concentrations différentes un composant à effet catalytique constitué par du ou des métaux nobles du groupe du platine.

2. Détecteur selon la revendication 1,
caractérisé en ce que
la matière de base céramique de la ou des couches de conversion (4, 6) est de l'oxyde de titane (TiO₂) et/ou de l'oxyde de zirconium (ZrO₂) et/ou de l'oxyde de silicium (SiO₂) et/ou de l'oxyde d'aluminium (Al₂O₃).

3. Détecteur selon la revendication 1,
caractérisé en ce que
la couche d'oxyde métallique (3) est en un oxyde métallique semi-conducteur notamment de l'oxyde d'indium (In₂O₃) ou de l'oxyde d'étain (SnO₂) auquel on mélange un élément de dopage augmentant la conductivité selon une concentration comprise entre 5 x 10 ⁻⁴ jusqu'à 5 x 10 ⁻² % molaire.

4. Détecteur selon la revendication 3,
caractérisé en ce que
l'élément de dopage est du tantale (Ta), du niobium (Nb), de l'antimoine (Sb), tungstène (W) et pour l'oxyde d'indium (In₂O₃) on utilise de l'étain (Sn), du titane (Ti) ou du cérium (Ce).

5. Détecteur selon la revendication 1,
caractérisé en ce que
la couche d'oxyde métallique (3) contient en outre au moins un élément de valence (2) du groupe principal II et/ou un élément de valence (3) du groupe principal III et/ou un élément de valence (3) choisi dans les terres rares, comme oxyde suivant une concentration comprise entre 0,001 jusqu'à 45 % en poids.

6. Détecteur selon la revendication 5,
caractérisé en ce que
l'élément supplémentaire est du magnésium (Mg), du baryum (Ba), du calcium (Ca), du strontium (Sr), du zinc (Zn) ou de l'aluminium à l'état d'oxyde d'aluminium (Al₂O₃)

7. Détecteur selon la revendication 1,
caractérisé en ce que
la couche d'oxyde métallique (3) contient du palladium (Pd) avec une addition de platine (Pt) et/ou de rhodium (Rh) et/ou un alliage de ceux-ci suivant une concentration comprise entre 0,001 jusqu'à 5 % molaire.

8. Détecteur selon la revendication 1,
caractérisé en ce que
la couche partielle de conversion (4) contient selon une concentration comprise entre 0,01 jusqu'à 20 % en poids de platine (Pt) et/ou de rhodium (Rh) et/ou de palladium (Pd) ou un alliage de ceux-ci.

9. Détecteur selon la revendication 1,
caractérisé en ce que
la couche partielle de conversion (4) reçoit une couche poreuse et sa face supérieure reçoit le dispositif de chauffage (5).

10. Détecteur selon la revendication 1,
caractérisé en ce que
la couche partielle de conversion (6) présente une teneur plus faible en métal noble que l'autre couche partielle de conversion (4).
